(19) Europäisches Patentamt
European Patent Office
Office européen des brevets

(11) **EP 2 296 004 A2**

(12) **EUROPEAN PATENT APPLICATION**

(43) Date of publication:
**16.03.2011 Bulletin 2011/11**

(51) Int Cl.:
**G01S 7/52** (2006.01)     **G01S 15/89** (2006.01)

(21) Application number: **10174361.5**

(22) Date of filing: **27.08.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **02.09.2009 KR 20090082505**

(71) Applicant: **Medison Co., Ltd.**
**Kangwon-do 250-870 (KR)**

(72) Inventor: **Ahn, Chi Young**
**135-851 Seoul (KR)**

(74) Representative: **Schmid, Wolfgang**
**Lorenz & Kollegen**
**Patentanwälte Partnerschaftsgesellschaft**
**Alte Ulmer Strasse 2**
**89522 Heidenheim (DE)**

(54) **Ultrasound system and method for providing an ultrasound spatial compound image considering steering angle**

(57)     Embodiments for providing an ultrasound spatial compound image are disclosed. In one embodiment, by way of non-limiting example, an ultrasound system comprises: an ultrasound data acquisition unit configured to transmit and receive ultrasound signals to and from a target object based on a plurality of steering angles corresponding to a plurality of frames to output a plurality of ultrasound data; and a processing unit in communication with the ultrasound data acquisition unit, the processing unit being configured to set a reference steering angle corresponding to each of the frames, calculate a steering angle corresponding to each of the frames by using a sequence based on the reference steering angle, perform scan conversion upon the ultrasound data to form the frames, and perform a spatial compound imaging upon images of spatially overlapped regions in the frames to form an ultrasound spatial compound image.

## FIG. 1

100

130 STORAGE UNIT

110 ULTRASOUND DATA ACQUISITION UNIT ↔ 120 PROCESSING UNIT ↔ 140 DISPLAY UNIT

**Description**

TECHNICAL FIELD

The present disclosure generally relates to ultrasound systems, and more particularly to providing an ultrasound spatial compound image based on steering angles, which are set by using a sequence, in an ultrasound system.

BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two or three-dimensional diagnostic images of internal features of an object (e.g., human organs).

The ultrasound system transmits and receives ultrasound signals to and from a target object to thereby form a 2D (two-dimensional) ultrasound image or a 3D (three-dimensional) ultrasound image. Various techniques have been studied to enhance resolution of the ultrasound image. Spatial compound imaging is known as one of such techniques.

The spatial compound imaging is an imaging technique for forming a single compounding image by combining ultrasound images obtained from multiple points and angles. That is, the ultrasound system electronically steers scanlines at different steering angles to thereby form a plurality of ultrasound images. The ultrasound system may then compound the ultrasound image to form an ultrasound spatial compound image.

The conventional ultrasound system forms an ultrasound spatial compound image by performing the spatial compound imaging upon images of spatially overlapped regions in the ultrasound images based on an ultrasound image formed without steering of the scanlines. This not only limits the number of ultrasound images needed to form the ultrasound spatial compound image, but also reduces quality of the ultrasound spatial compound image. This is because the boundary line of the ultrasound spatial compound image, the scanlines of which are steered, is marked on the compound image.

Further, the conventional ultrasound system calculates the steering angles of scanlines based on a common point by extending the scanlines to the back of an ultrasound probe, thereby obtaining the ultrasound images needed to form an ultrasound spatial compound image. This additionally requires hardware or software resources for producing a steering angle.

SUMMARY

Embodiments for providing a plurality of slice images in an ultrasound system are disclosed herein. In one embodiment, by way of non-limiting example, an ultrasound system comprises: an ultrasound data acquisition unit configured to transmit and receive ultrasound signals to and from a target object based on a plurality of steering angles corresponding to a plurality of frames to output a plurality of ultrasound data; and a processing unit in communication with the ultrasound data acquisition unit, the processing unit being configured to set a reference steering angle corresponding to each of the frames, calculate a steering angle corresponding to each of the frames by using a sequence based on the reference steering angle, perform scan conversion upon the ultrasound data to form the frames, and perform a spatial compound imaging upon images of spatially overlapped regions in the frames to form an ultrasound spatial compound image.

In another embodiment, there is provided a method of providing an ultrasound spatial compound image, comprising: a) setting a reference steering angle corresponding to each of a plurality of frames; b) calculating a plurality of steering angles corresponding to the frames by using a sequence based on the reference steering angle; c) acquiring a plurality of ultrasound data based on the steering angles; d) performing scan conversion upon the ultrasound data to form the frames; and e) performing a spatial compound imaging upon images of spatially overlapped regions in the frames to form an ultrasound spatial compound image.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1        is a block diagram showing an illustrative embodiment of an ultrasound system.
FIG. 2        is a block diagram showing an illustrative embodiment of an ultrasound data acquisition unit.

FIG. 3        is a flow chart showing a process of forming an ultrasound spatial compound image considering steering angles

FIGS. 4 to 6      are schematic diagrams showing examples of setting a plurality of scanlines.

FIG. 7        is a schematic diagram showing an example of performing scan conversion with a linear ultrasound probe.

FIG. 8        is a schematic diagram showing an example of performing the scan conversion with a convex ultrasound probe.

FIG. 9        is a schematic diagram showing an example of performing the scan conversion with a 2-D array ultrasound probe.

DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

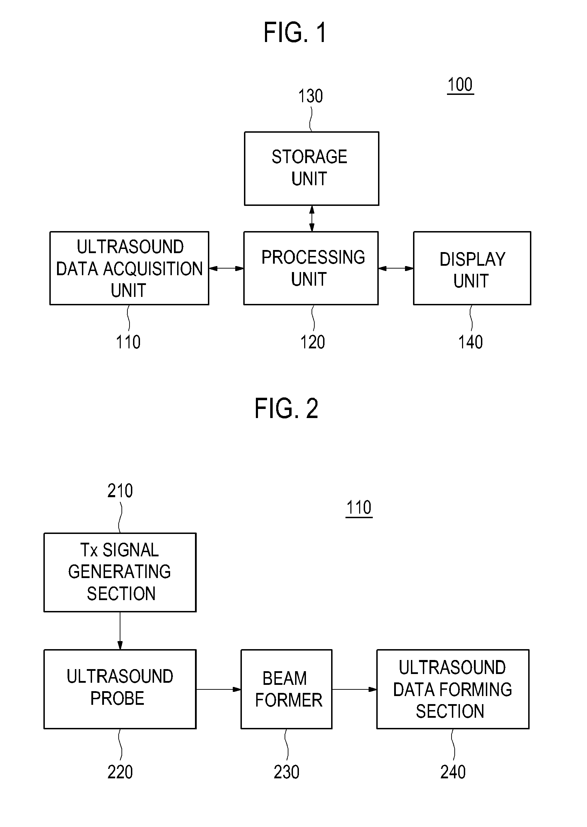

FIG. 1 is a block diagram showing an illustrative embodiment of an ultrasound system 100. Referring to FIG. 1, the ultrasound system 100 may include an ultrasound data acquisition unit 110. The ultrasound data acquisition unit 110 may be configured to transmit and receive ultrasound signals to and from a target object to output ultrasound data.

FIG. 2 is a block diagram showing an illustrative embodiment of the ultrasound data acquisition unit 110. Referring to FIG. 2, the ultrasound data acquisition unit 110 may include a transmit (Tx) signal generating section 210, an ultrasound probe 220, a beam former 230 and an ultrasound data forming section 240.

The Tx signal generating section 210 may be configured to generate Tx signals. In one embodiment, the Tx signal generating section 210 may generate a plurality of Tx signals with different Tx pattern such that scan-lines are steered at different steering angles. Thus, a plurality of frames $P_i$ ($1 \leq i \leq N$) corresponding to the respective steering angles may be obtained. The frame may include a brightness mode (B mode) image. However, it should be noted herein that the frame may not be limited thereto.

The ultrasound probe 220 may include a plurality of elements (not shown) for reciprocally converting between ultrasound signals and electrical signals. The ultrasound probe 220 may be configured to transmit ultrasound signals into the target object in response to the Tx signals provided from the Tx signal generating section 210. The ultrasound probe 220 may further receive ultrasound echo signals reflected from the target object to thereby form received signals. The received signals may be analog signals. The ultrasound probe 220 may include a linear probe, a convex probe and the like. However, it should be noted herein that the ultrasound probe 220 may not be limited thereto.

The beam former 230 may be configured to convert the received signals provided from the ultrasound probe 220 into digital signals. The beam former 230 may further apply delays to the digital signals in consideration of distances between the elements and focal points to thereby output digital receive-focused signals.

The ultrasound data forming section 240 may be configured to form ultrasound data corresponding to each of frames $P_i$ ($1 \leq i \leq N$) based on the digital receive-focused signals provided from the beam former 230. The ultrasound data may be radio frequency (RF) data. However, it should be noted herein that the ultrasound data may not be limited thereto. The ultrasound data forming section 240 may further perform various signal processing (e.g., gain adjustment) to the digital receive-focused signals. Referring back to FIG. 1, the ultrasound system 100 may further include a processing unit 120 in communication with the ultrasound data acquisition unit 110.

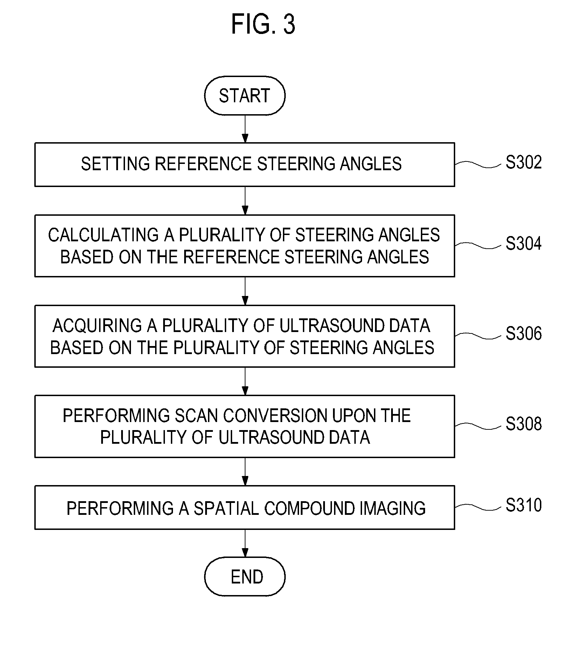

FIG. 3 is a flow chart showing a process of forming an ultrasound spatial compound image in consideration of steering angles. The processing unit 120 may be configured to set a reference steering angle (view angle) for each of frames $P_i$ ($1 \leq i \leq K$), at step S302 in FIG. 3. The reference steering angle may represent a steering angle of the first scanline $S_1$ or the last scanline $S_n$ in a plurality of scanlines $S_i$ ($1 \leq i \leq n$), and may be set differently according to the frames.

The processing unit 120 may be configured to calculate steering angles of the scanlines $S_i$ ($1 \leq i \leq n$) based on the reference steering angle, at step S304 in FIG. 3. Thus, the ultrasound data acquisition unit 110 may output the plurality of ultrasound data corresponding to the plurality of frames $P_i$ ($1 \leq i \leq N$) based on the calculated steering angles provided from the processing unit 120.

The steering angles of the scanlines $S_i$ ($1 \leq i \leq n$) may be calculated by using an arithmetic sequence, a geometric sequence, a combination thereof and other sequences based on the reference steering angle. For example, a steering angle $\theta_i$ of $S_i$ may be calculated by using arithmetic sequence as the following equation.

$$\theta_i = \theta_1 + (i\text{-}1) \times \theta_d \qquad\qquad (1)$$

In the above equation, $\theta_1$ is the steering angle of the first scanline $S_1$ and $\theta_d$ is a common difference. The common difference may be produced as the following equation.

$$\theta_n = \theta_1 + (n-1) \times \theta_d$$

$$\theta_n = -\theta_1$$

$$\theta_d = (2 \times \theta_n)/(n-1) \qquad (2)$$

In the above equation, $\theta_n$ is the steering angle of the last scanline $S_n$.

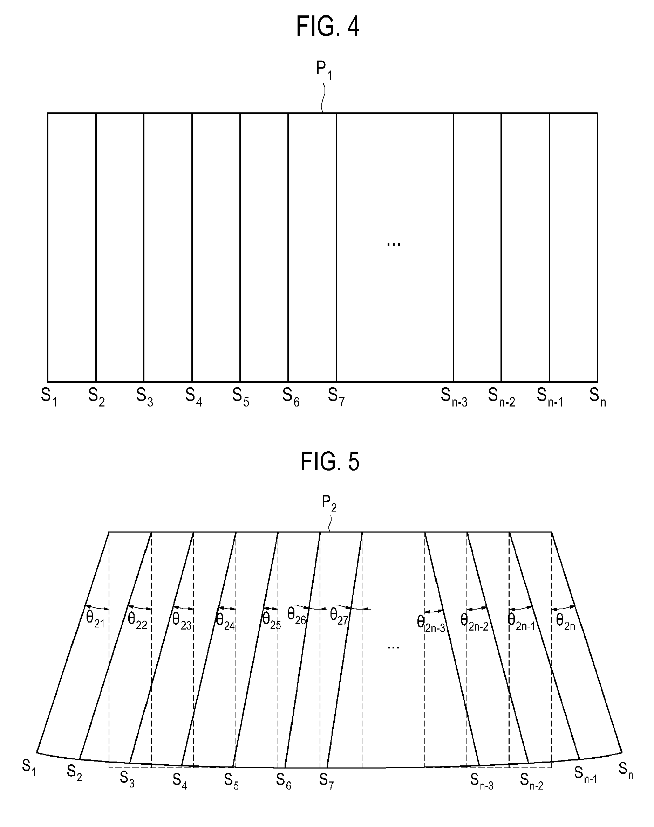

In one embodiment, the processing unit 120 may set a first reference steering angle (i.e., 0°) for the frame $P_1$, as shown in FIG. 4. That is, the processing unit 120 may set the steering angle 0° of each of the scanline $S_i$ ($1 \leq i \leq n$) based on the first reference steering angle 0°.

The processing unit 120 may further set a second reference steering angle $\theta_{21}$ of the scanline $S_1$ for the frame $P_2$, as shown in FIG. 5. The processing unit 120 may further calculate steering angles $\theta_{22}$ to $\theta_{2n}$ corresponding to the scanlines $S_2$ to $S_n$ through the equations 1 and 2 based on the second reference steering angle $\theta_{21}$.

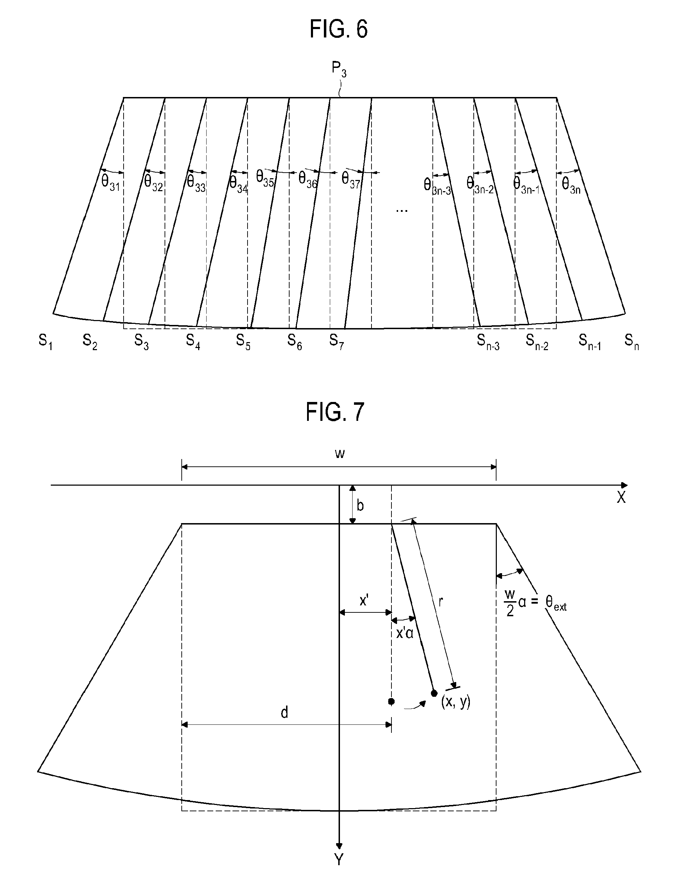

The processing unit 120 may further set a third reference steering angle $\theta_{31}$ of the scanline $S_1$ for the frame $P_3$, as shown in FIG. 6. The processing unit 120 may further calculate steering angles $\theta_{32}$ to $\theta_{3n}$ corresponding to scanlines $S_1$ to $S_n$ through the equations 1 and 2 based on the third reference steering angle $\theta_{31}$.

The processing unit 120 may further set the reference steering angles $\theta_{i1}$ corresponding to the frames $P_i$ ($4 \leq i \leq K$), and calculate the steering angles corresponding to the scanlines $S_1$ to $S_n$ through the equations 1 and 2 based on the reference steering angles $\theta_{i1}$, as mentioned above.

When the plurality of ultrasound data acquired by the ultrasound data acquisition unit 110 are provided at step S306 in FIG 3, the processing unit 120 may be configured to perform scan conversion upon the ultrasound data provided from the ultrasound data acquisition unit 110 to form the plurality of frames (i.e., ultrasound images), at step S308. The processing unit 120 may perform the scan conversion upon the ultrasound data based on a type of the ultrasound probe 220.

In an embodiment, if the ultrasound probe 220 is a linear probe, then the processing unit 120 may perform the scan conversion upon the ultrasound data as the following equation.

$$d = x' + \frac{w}{2}, \quad r = \sqrt{(x-x')^2 + (y-b)^2}$$

$$x' = \sqrt[3]{A} + \sqrt[3]{B}, \quad A = \frac{-q + \sqrt{q^2 + 4p^3}}{2}, \quad B = \frac{-q - \sqrt{q^2 + 4p^3}}{2}$$

$$p = \frac{1 + (y-b)\alpha}{(y-b)\alpha^3}, \quad q = -\frac{3x}{(y-b)\alpha^3}, \quad \alpha = \frac{2\theta_{ext}}{w} \qquad (3)$$

In the above equation, $\theta_{ext}$ represents the reference steering angle as shown in FIG. 7, w represents a width of frame, (x, y) represents coordinate of an ultrasound image converted with scan conversion and b represents image offset and may be generally ignored.

That is, the processing unit 120 may extract the ultrasound data of (d, r) coordinate corresponding to (x, y) coordinate of the ultrasound image through the above equation to thereby perform the scan conversion upon the ultrasound data.

In another embodiment, if the ultrasound probe 220 is the convex probe, then the processing unit 120 may perform the scan conversion upon the ultrasound data as the following equation.

$$\sigma = \frac{\pi}{2} + \frac{\gamma}{\left(1 - \frac{b}{l}\right)\alpha + 1} \quad , \quad r = \sqrt{b^2 + l^2 - 2bl\cos(\gamma - \theta)}$$

$$\alpha = \frac{\theta_{ext} - \theta_{org}}{\theta_{org}} \quad , \quad \gamma = \tan^{-1}\left(\frac{x}{y}\right), \quad l = \sqrt{x^2 + y^2} \quad , \quad \theta = \sigma - \frac{\pi}{2} \qquad (4)$$

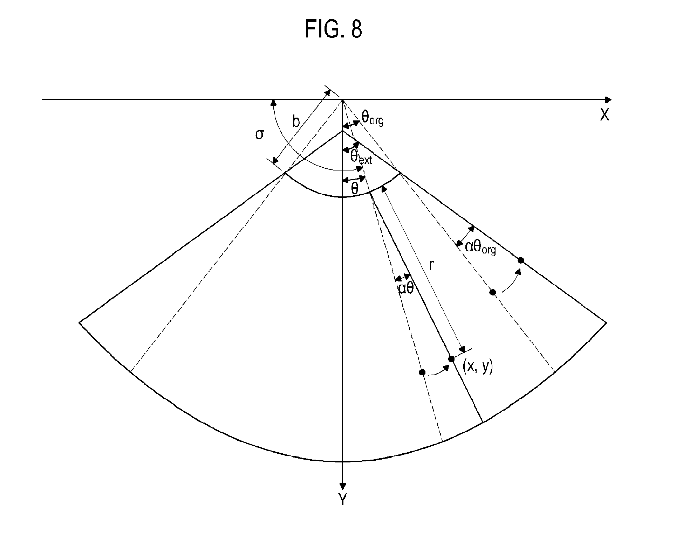

In the above equation, $\theta_{ext}$ represents the reference steering angle as shown in FIG. 8, $\theta_{org}$ represents a view angle of a frame whose scanlines are not steered, and b represents image offset and may be generally ignored.

That is, the processing unit 120 may extract the ultrasound data of ($\sigma$, r) coordinate corresponding to (x, y) coordinate of the ultrasound image using the above equation to thereby perform the scan conversion upon the ultrasound data.

The processing unit 120 may be configured to perform spatial compound imaging upon the plurality of ultrasound images to thereby form an ultrasound spatial compound image, at step S310 in FIG. 3. In one embodiment, the processing unit 120 may perform the spatial compound imaging upon images of spatially overlapped regions in the plurality of frames (ultrasound images) $P_i$ ($1 \leq i \leq K$) to form an ultrasound spatial compound image. The processing unit 120 may perform the spatial compound imaging by using arithmetic mean, geometric mean or harmonic mean of the pixel values of each of the ultrasound images. Also, the processing unit 120 may apply different weights to the plurality of frames $P_i$ ($1 \leq i \leq K$) and may perform the spatial compound imaging upon the plurality of frames $P_i$ ($1 \leq i \leq K$) with the applied weights. For example, the processing unit 120 may apply a maximum weight value to the frame $P_1$ of a minimum steering angle and apply a minimum weight value to the frame of a maximum steering angle.

Referring back to FIG. 1, the ultrasound system 100 may further include a storage unit 130. The storage unit 130 may store the plurality of ultrasound data provided from the ultrasound data acquisition unit 110. The storage unit 130 may further store the plurality of frames $P_i$ ($1 \leq i \leq K$) provided from the processing unit 120.

The ultrasound system 100 may further include a display unit 140. The display unit 140 may display the ultrasound spatial compound image provided from the processing unit 120. The display unit 140 may further display the plurality of frames $P_i$ ($1 \leq i \leq K$) provided from the processing unit 120.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements within the scope of the disclosure, the drawings and the appended claims. In addition to variations and modifications in the component parts and/or arrangements, alternative uses will also be apparent to those skilled in the art.

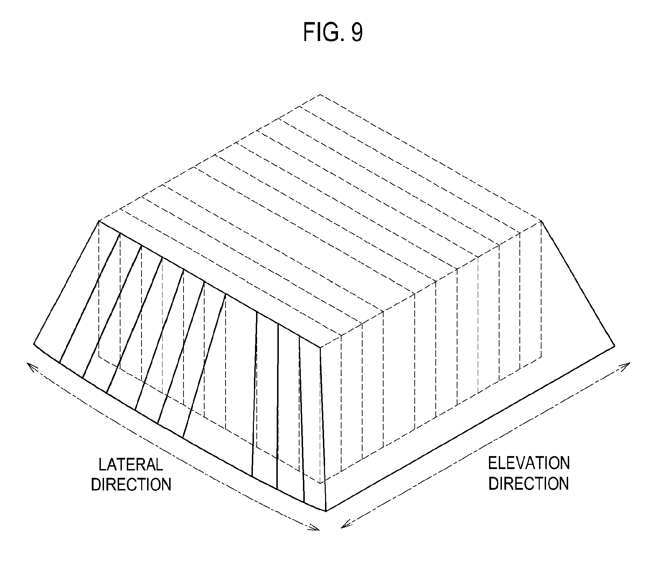

For example, in above embodiment, the steering angle of the scanlines may be set when the ultrasound probe 220 is a one-dimensional (1D) array probe. In another embodiment, the reference steering angle of the scanlines may be established using the sequences mentioned above based on scanline which is not steered to the lateral direction where the ultrasound probe 220 is a 2-D array probe or 3-D array, as shown in FIG. 9. A steering angle of scanline may be established using sequences mentioned above based on frame which is not steered to the elevation direction. Further, a 3-D compound image may be formed by compounding a plurality of volume data with different view angles.

The terminology used herein is for the purpose of describing particular embodiments only and is not intended to limit the present invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements and/or components, but do not preclude the presence or addition of one or more other features, integers, steps, operations, elements, components and/or groups thereof.

**Claims**

1.  An ultrasound system, comprising:

    an ultrasound data acquisition unit configured to transmit and receive ultrasound signals to and from a target object based on a plurality of steering angles corresponding to a plurality of frames to output a plurality of ultrasound data; and

a processing unit in communication with the ultrasound data acquisition unit,

the processing unit being configured to set a reference steering angle corresponding to each of the frames, calculate a steering angle corresponding to each of the frames by using a sequence based on the reference steering angle,

perform scan conversion upon the ultrasound data to form the frames, and

perform a spatial compound imaging upon images of spatially overlapped regions in the frames to form an ultrasound spatial compound image.

**2.** The ultrasound system of Claim 1, wherein the reference steering angle is the steering angle of a first scanline or a last scanline in the scanlines.

**3.** The ultrasound system of Claim 1, wherein the sequence is selected from the group consisting of an arithmetic sequence, a geometric sequence and a combination thereof.

**4.** The ultrasound system of Claim 1, wherein the ultrasound data acquisition unit includes an ultrasound probe.

**5.** The ultrasound system of Claim 4, wherein the processing unit is configured to perform the scan conversion upon the ultrasound data based on a type of the ultrasound probe.

**6.** The ultrasound system of Claim 1, wherein the processing unit is configured to perform the spatial compound imaging by using an arithmetic mean, a geometric mean or a harmonic mean.

**7.** The ultrasound system of Claim 1, wherein the processing unit is configured to apply different weights to the frames and perform the spatial compound imaging upon the frames with the applied weights.

**8.** A method of providing an ultrasound spatial compound image, comprising:

a) setting a reference steering angle corresponding to each of a plurality of frames;

b) calculating a plurality of steering angles corresponding to the frames by using a sequence based on the reference steering angle;

c) acquiring a plurality of ultrasound data based on the steering angles;

d) performing scan conversion upon the ultrasound data to form the frames; and

e) performing a spatial compound imaging upon images of spatially overlapped regions in the frames to form an ultrasound spatial compound image.

**9.** The method of Claim 8, wherein the reference steering angle is the steering angles of a first scanline or a last scanline in the plurality of scanlines.

**10.** The method of Claim 8, wherein the sequence is selected from the group consisting of an arithmetic sequence, a geometric sequence, and a combination thereof.

**11.** The method of Claim 8, wherein the step d) comprises:

performing the scan conversion upon the ultrasound data based on a type of ultrasound probe.

**12.** The method of Claim 8, wherein the step e) comprises:

performing the spatial compound imaging by using an arithmetic mean, a geometric mean of a harmonic mean.

**13.** The method of Claim 8, wherein the step e) comprises:

applying different weights to the frames; and

performing the spatial compound imaging upon the frames with the applied weights.

# FIG. 1

<u>100</u>

130

```
┌─────────────┐
│   STORAGE   │
│    UNIT     │
└─────────────┘
       ↕
```

| ULTRASOUND DATA ACQUISITION UNIT | ↔ | PROCESSING UNIT | ↔ | DISPLAY UNIT |
|---|---|---|---|---|

110                120                140

# FIG. 2

210

<u>110</u>

```
┌──────────────┐
│  Tx SIGNAL   │
│  GENERATING  │
│   SECTION    │
└──────────────┘
       ↓
```

| ULTRASOUND PROBE | → | BEAM FORMER | → | ULTRASOUND DATA FORMING SECTION |
|---|---|---|---|---|

220                230                240

# FIG. 3

START

|  |  |
| --- | --- |
| SETTING REFERENCE STEERING ANGLES | S302 |

|  |  |
| --- | --- |
| CALCULATING A PLURALITY OF STEERING ANGLES BASED ON THE REFERENCE STEERING ANGLES | S304 |

|  |  |
| --- | --- |
| ACQUIRING A PLURALITY OF ULTRASOUND DATA BASED ON THE PLURALITY OF STEERING ANGLES | S306 |

|  |  |
| --- | --- |
| PERFORMING SCAN CONVERSION UPON THE PLURALITY OF ULTRASOUND DATA | S308 |

|  |  |
| --- | --- |
| PERFORMING A SPATIAL COMPOUND IMAGING | S310 |

END

## FIG. 4

$P_1$

$S_1$  $S_2$  $S_3$  $S_4$  $S_5$  $S_6$  $S_7$   ...   $S_{n-3}$  $S_{n-2}$  $S_{n-1}$  $S_n$

## FIG. 5

$P_2$

$\theta_{21}$  $\theta_{22}$  $\theta_{23}$  $\theta_{24}$  $\theta_{25}$  $\theta_{26}$  $\theta_{27}$   ...   $\theta_{2n-3}$  $\theta_{2n-2}$  $\theta_{2n-1}$  $\theta_{2n}$

$S_1$  $S_2$  $S_3$  $S_4$  $S_5$  $S_6$  $S_7$   $S_{n-3}$  $S_{n-2}$  $S_{n-1}$  $S_n$

## FIG. 6

P_3

$\theta_{31}$  $\theta_{32}$  $\theta_{33}$  $\theta_{34}$  $\theta_{35}$  $\theta_{36}$  $\theta_{37}$  $\theta_{3n-3}$  $\theta_{3n-2}$  $\theta_{3n-1}$  $\theta_{3n}$

...

$S_1$   $S_2$   $S_3$   $S_4$   $S_5$   $S_6$   $S_7$   $S_{n-3}$   $S_{n-2}$   $S_{n-1}$   $S_n$

## FIG. 7

w

X

b

x'

r

$\frac{w}{2}\alpha = \theta_{ext}$

x'α

d

(x, y)

Y

# FIG. 8

FIG. 9

LATERAL
DIRECTION

ELEVATION
DIRECTION